# EUROPEAN PATENT APPLICATION

(11) **EP 1 484 311 A1**
(43) Date of publication of application: **08.12.2004**
(21) Application number: 03705268.5
(22) Date of filing: 18.02.2003
(51) Int. Cl.: C07C 67/08, C07C 69/68

(54) **PROCESS FOR PRODUCING CHAIN OLIGOLACTIC ACID ESTER**

(30) Priority: 19.02.2002 JP 2002042009
(71) Applicant: AMATO PHARMACEUTICAL PRODUCTS, LTD., Fukuchiyama-shi, Kyoto 620-0932 (JP)
(72) Inventor: WATANABE, Mikio, Hadano-shi, Kanagawa 257-0002 (JP); MURAKAMI, Masahiro, Osaka-shi, Osaka 547-0026 (JP)
(74) Representative: Sternagel, Fleischer, Godemeyer & Partner
(86) International application number: PCT/JP2003/001697
(87) International publication number: WO 2003/070684

(57) **Abstract**

An object of the present invention is to establish a method for directly synthesizing an oligolactic acid ester having a specific chain length as a single compound. According to the present invention, there is provided a method for producing a compound represented by formula (1): 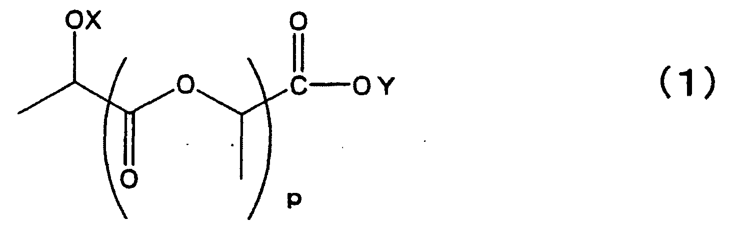 (in the formula (1), X represents a hydrogen atom or a protective group for a hydroxyl group, Y represents a hydrogen atom or a lower alkyl group, and p represents an integer of 2 to 19, satisfying m+n+1=p),
which comprises reacting a compound represented by formula (2) defined in the specification with a compound represented by formula (3) defined in the specification.

## Description

### TECHNICAL FIELD

The present invention relates to a method for producing a linear oligolactic acid ester. More particularly, the present invention relates to a method for producing a purified linear oligolactic acid ester which is composed of an single compound.

### BACKGROUND ART

A mixture of cyclic and/or linear poly L-lactic acids having condensation degrees of 3-19 is reported to be useful as an anti-malignant tumor agent (Japanese Patent Laid-open Publication Nos. 9-227388 and 10-130153) and a QOL improving agent for a cancer patient (JP Application No. 11-39894; Journal of Japan Society of Clinical Oncology, vol. 33, No. 3, p493). Furthermore, a mixture of cyclic and/or linear poly L-lactic acids having condensation degree of 3-19 is known to have a blood sugar reducing action so that it is useful as a medicament for prevention and/or treatment of diabetes or a diabetic complication (JP Application No. 11-224883), and is further proved to be useful for suppressing over-appetite, promoting basal metabolism, and improving and/or preventing obesity.

As described above, the mixture of cyclic and/or linear poly L-lactic acids having condensation degree of 3-19 has been demonstrated to exhibit various pharmacological effects, and is expected to be developed as a medicament in future. In order to develop an oligolactic acid as a medicament, it is desirable to isolate it as a single compound having a specific chain length instead of a mixture consisting of poly lactic acids having respective different chain lengths. In order to isolate a polylactic acid having a specific chain length as a single compound, an approach to synthesize a linear oligolactic acid ester having a condensation degree of 3 to 20, of which the terminus carboxyl group has been esterified, has been carried out (JP Application No. 2001-69766). However, the production method described in JP Application No. 2001-69766 is a method for producing a mixture of linear oligolactic acid esters having condensation degrees of 3-20, from which an oligolactic acid ester having a specific chain length is then purified. Therefore, there has not yet been any report on synthesizing directly a polylactic acid having a specific chain length as a single compound.

### DISCLOSURE OF THE INVENTION

A problem to be solved by the present invention is to establish a method for directly synthesizing an oligolactic acid ester having a specific chain length as a single compound.

The present inventors made diligent studies to solve the above problem, and as a result, they have found that a lactic acid oligomer having certain chain length can be synthesized by reacting a lactic acid oligomer of which hydroxyl group has been protected with lactic acid or a lactic acid oligomer of which carboxyl group has been protected. Further, the present inventors have found that the protective groups for the hydroxyl group and the carboxyl group can be selectively deprotected respectively in the obtained lactic acid oligomer having certain chain length. The present invention has been completed based on these findings.

Namely, the present invention provides a method for producing a compound represented by formula (1): 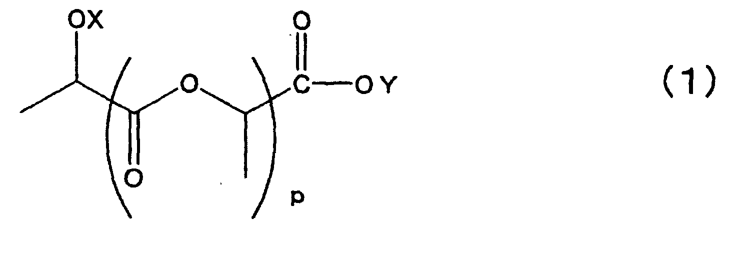 (in the formula (1), X represents a hydrogen atom or a protective group for a hydroxyl group, Y represents a hydrogen atom or a lower alkyl group, and p represents an integer of 2 to 19, satisfying m+n+1=p),
which comprises reacting a compound represented by formula (2) with a compound represented by formula (3): 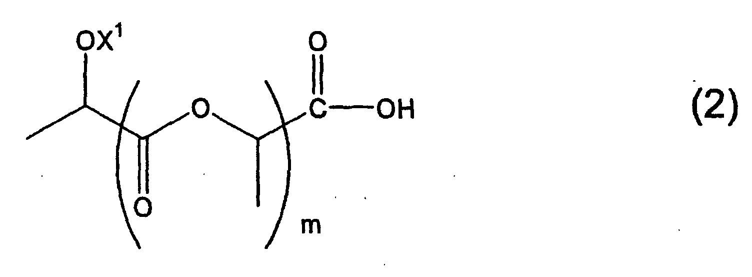 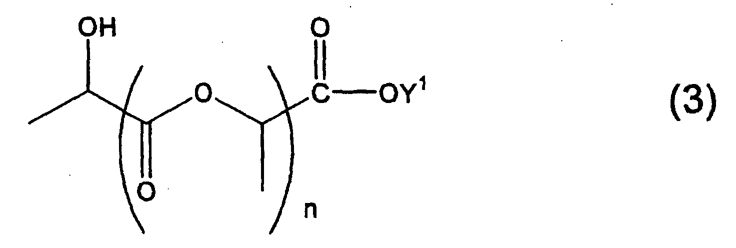 (in the formula (2), X¹ represents a protective group for a hydroxyl group, and m represents an integer of 0-18; and in the formula (3), Y¹ represents a lower alkyl group, and n represents an integer of 0-18).

Another aspect of the invention provides a method for deprotecting selectively a protective group for a hydroxyl group in the formula (1): 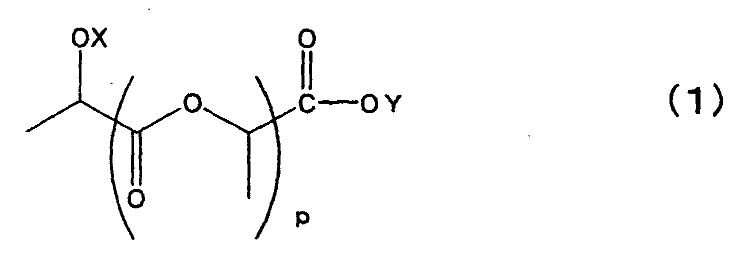 wherein X represents a protective group for a hydroxyl group, Y represents a lower alkyl group, and m represents an integer of 1 to 19,
wherein the compound represented by the formula (1) is treated with hydrofluoric acid.

Further another aspect of the present invention provides a method for deprotecting selectively a protective group for a carboxyl group in the formula (1): 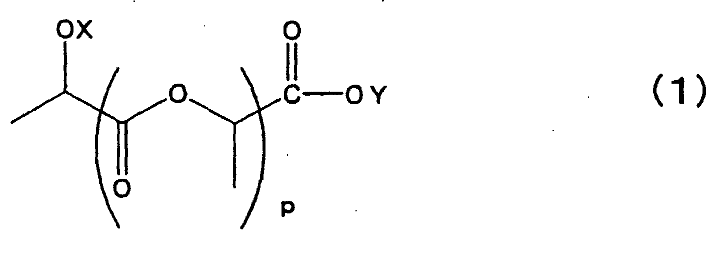 wherein X represents a protective group for a hydroxyl group, Y represents a lower alkyl group, and m represents an integer of 1 to 19,
wherein the compound represented by the formula (1) is treated with trifluoroacetic acid/methylene chloride.

Preferably in the present invention, X is a trialkylsilyl group and Y is a tert-butyl group.

### BEST MODE FOR CARRYING OUT THE INVENTION

Embodiments and practice methods of the invention will be described in detail below.

The invention relates to a method for producing a compound represented by formula (1): 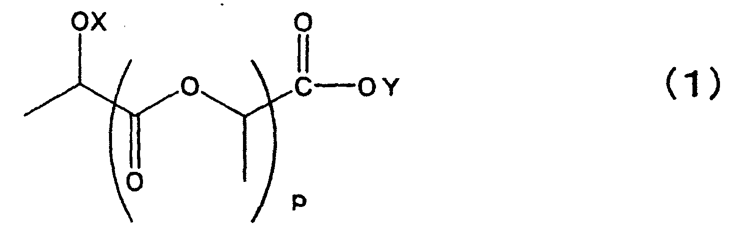 (in the formula (1), X represents a hydrogen atom or a protective group for a hydroxyl group, Y represents a hydrogen atom or a lower alkyl group, and p represents an integer of 2 to 19, satisfying m+n+1=p),
which comprises reacting a compound represented by formula (2) with a compound represented by formula (3): 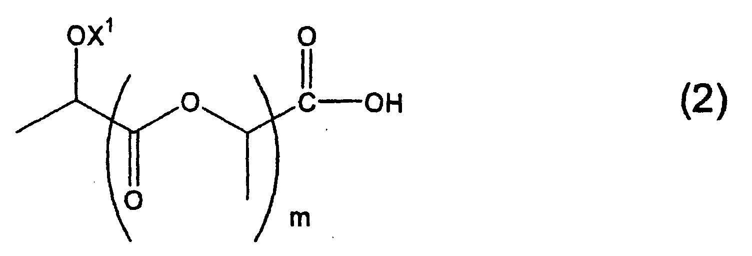 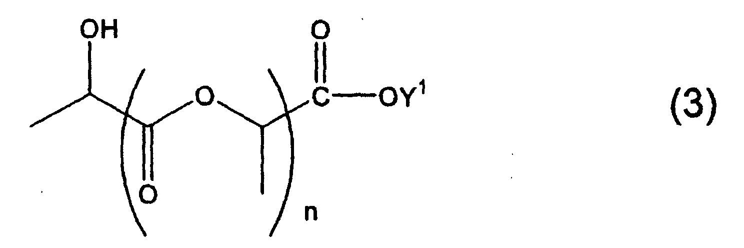 (in the formula (2), X¹ represents a protective group for a hydroxyl group, and m represents an integer of 0-18; and in the formula (3), Y¹ represents a lower alkyl group, and n represents an integer of 0-18).

In the present invention, the protective group for a hydroxyl group represented by X or X¹ is not particularly limited, and may be suitably selected by a person skilled in the art. Specific examples of the protective group for a hydroxyl group include those described below:
(Ether type)
   methyl group, methoxymethyl group, methylthiomethyl group, benzyloxymethyl group, t-butoxymethyl group, 2-methoxyethoxymethyl group, 2,2,2-trichloroethoxymethyl group, bis(2-chloroethoxy)methyl group, 2-(trimethylsilyl)ethoxymethyl group, tetrahydropyranyl group, 3-bromotetrahydropyranyl group, tetrahydrothiopyranyl group, 4-methoxytetrahydropyranyl group, 4-methoxytetrahydrothiopyranyl group, 4-methoxytetrahydrothiopyranyl S,S-dioxide group, tetrahydrofuranyl group, tetrahydrothiofuranyl group;
   1-ethoxyethyl group, 1-methyl-1-methoxyethyl group, 1-(isopropoxy)ethyl group, 2,2,2-trichloroethyl group, 2-(phenylserenyl)ethyl group, t-butyl group, allyl group, cinnamyl group, p-chlorophenyl group, benzyl group, p-methoxybenzyl group, o-nitrobenzyl group, p-nitrobenzyl group, p-halobenzyl group, p-cyanobenzyl group, 3-methyl-2-picolyl N-oxide group, diphenylmethyl group, 5-dibenzosuberyl group, triphenylmethyl group, a-naphthyldiphenylmethyl group, p-methoxyphenyldiphenylmethyl group, p-(p'-bromophenacyloxy)phenyldiphenylmethyl group, 9-anthryl group, 9-(9-phenyl)xanthenyl group, 9-(9-phenyl-10-oxo)anthryl group, and benzisothiazolyl S,S-dioxide group;
   trimethylsilyl group, triethylsilyl group, isopropyldimethylsilyl group, t-butyldimethylsilyl group (TBDMS group), (triphenylmethyl)dimethylsilyl group, t-butyldiphenylsilyl group, methyldiisopropylsilyl group, methyl di-t-butylsilyl group, tribenzylsilyl group, tri-p-xylylsilyl groupl, triisopropylsilyl group, and triphenylsilyl group;
(Ester type)
   formate, benzoyl formate, acetate, chloroacetate, dichloroacetate, trichloroacetate, trifluoroacetate, methoxyacetate, triphenylmethoxyacetate, phenoxyacetate, p-chlorophenoxyacetate, 2,6-dichloro-4-methylphenoxyacetate, 2,6-dichloro-4-( 1,1,3,3-tetramethylbutyl)phenoxyacetate, 2,4-bis(1,1-dimethylpropyl)phenoxyacetate, chlorodiphenylacetate, p-P-phenylacetate, 3-phenylpropionate, 3-benzoylpropionate, isobutylate, monosuccinoate, 4-oxopentanoate, pivaloate, adamantoate, crotonate, 4-methoxycrotonate, (E)-2-methyl-2-butenoate, benzoate, o-(dibromomethyl)benzoate, o-(methoxycarbonyl)benzoate, p-phenylbenzoate, 2,4,6-trimethylbenzoate, p-P-benzoate, and a-naphthoate;
(Carbonate type)
   methylcarbonate, ethylcarbonate, 2,2,2-trichloroethylcarbonate, isobutylcarbonate, vinylcarbonate, allylcarbonate, cinnamylcarbonate, p-nitrophenylcarbonate, benzylcarbonate, p-methoxybenzylcarbonate, 3,4-dimethoxybenzylcarbonate, o-nitrobenzylcarbonate, p-nitrobenzylcarbonate, and S-benzylthiocarbonate; and
(others)
   N-phenylcarbamate, N-imidazolylcarbamate, borate, nitrate, N,N,N',N'-tetramethylphosphorodiamidate, and 2,4-dinitrophenylsulfenate.

A method for introducing and removing a protective group as described above are known to a person skilled in the art, and described in, for example, Teodora, W. Green, Protective Groups in Organic Synthesis, John & Wiley & Sons Inc. (1981) etc.

For the protective group for a hydroxyl group, a protective group which can be selectively introduced to the hydroxyl group, and a protective group which can be selectively deprotected from the hydroxyl group to which the protective group has been introduced, is preferably used. As such protective group for a hydroxyl group, a protective group of the ether type can be mentioned among those described above. Examples thereof particularly include a protective group of silylether type such as trimethylsilyl group, triethylsilyl group, isopropyldimethylsilyl group, t-butyldimethylsilyl group (TBDMS group), (triphenylmethyl)dimethylsilyl group, t-butyldiphenylsylyl group, methyldiisopropylsilyl group, methyl di-t-butylsilyl group, tribenzylsilyl group, tri-p-xylylsilyl group, triisopropylsilyl group, and triphenylsilyl group. The most preferred example is t-butyldimethylsilyl group (TBDMS group).

In the present invention, a lower alkyl group represented by Y or Y' is used as the protective group for a carboxyl group. The carbon number of the lower alkyl group represented by Y or Y' is not particularly limited, but generally 1-10, preferably 1-6, and more preferably 1-4. The chain type of the lower alkyl group is not particularly limited, and may be a linear, branched or cyclic chain, or any combination of them. Specific examples of the lower alkyl group include methyl group, ethyl group, propyl group, isopropyl group, cyclopropyl group, butyl group, isobutyl group, sec-butyl group, tert-butyl group, cyclopropylmethyl group, cyclobutyl group, pentyl group and hexyl group. The particularly preferred example of the lower alkyl group represented by Y or Y' is tert-butyl group.

In the present invention, m represents an integer of 0-18, and n represents an integer of 0-18. In the compound of formula (1) which is an object compound to be synthesized, p represents an integer of 2 to 19. Further, the values of m and n are selected to satisfy m+n+1=p.

The values of m, n and p correspond to the number of lactic acid unit. For example, the value of 0 gives a lactic acid monomer, 1 gives a lactic acid dimer, 2 gives a lactic acid trimer and, similarly, 19 gives a lactic acid eicosamer.

The production method of the present invention can provide an oligolactic acid ester having a specific chain length (i.e., a compound having certain p value in the formula (1)) as a compound having a single chemical structure, instead of a mixture containing oligolactic esters having different chain lengths.

When X is a hydrogen atom in the formula (1), the compound can exist as a metal salt. Such metal salt includes an alkali metal salt such as sodium salt and potassium salt, an alkali-earth metal salt such as magnesium salt and calcium salt, aluminum salt, zinc salt, etc. Further, various types of a hydrate, a solvate and a crystal forms of the compound of the formula (1) are provided, and all of them are included within the present invention.

Since the compound of formula (1) produced by the method of the present invention contains an asymmetric carbon, there are stereoisomers. All the possible isomers and a mixture containing two or more types of the isomers at an arbitrary ratio are also included within the present invention. Therefore, the compound produced by the present invention includes a mixture of various optical isomers such as an optically active substance, a racemic body and a diastereomer, and the isolated single substances thereof.

The configuration of the compound produced by the method of the invention depends on that of a lactic acid unit in a compound used as the starting material. That is, use of the L form, the D form, or a mixture thereof as the lactic acid unit in a starting material compound gives the compound of the invention in various configurations. In the present invention, as the configuration of the lactic acid unit, the L form is used preferably.

The compound represented by the formula (2) used in the present invention is a compound which is produced by protecting a hydroxyl group of lactic acid or a lactic acid oligomer. For example, a compound of m=1 among the compound represented by the formula (2) can be produced by protecting a hydroxyl group of lactoyl lactic acid with a protective group. Specifically, a solution of lactoyl lactic acid (obtained by ring-opening lactide) in DMF is added with a solution of t-butyldimethyl chlorosilane (TBDMSCl) and imidazole in DMF, followed by stirring to react. After the completion of the reaction, t-butyldimethylsiloxylactoyl lactic acid t-butyldimethylsiloxy ester can be obtained by a common purification and isolation. Further, the t-butyldimethylsiloxylactoyl lactic acid t-butyldimethylsiloxy ester is dissolved in a solvent containing methanol, dropped with an aqueous potassium carbonate solution, followed by stirring to react. After the reaction, t-butyldimethylsiloxylactoyl lactic acid, which is lactoyl lactic acid having a protected hydroxyl group, is obtained by common purification and isolation.

The compound represented by the formula (3) used in the present invention is a compound which is produced by protecting a carboxyl group of lactic acid or a lactic acid oligomer.

For example, a compound of n=0 among the compound represented by the formula (3) can be produced by protecting a carboxyl group of lactic acid with a protective group. Specifically, to a solution of 2-acetoxy propionic acid in methylene chloride is added a solution of tert-butyl acetate in methylene chloride, and further perchloric acid is added thereto, followed by stirring. After the reaction, tert-butyl 2-hydroxy propionate is obtained by common purification and isolation.

Further, a compound of n=1 among the compound represented by the formula (3) can be produced by protecting a carboxyl group of lactoyl lactic acid with a protective group. Specifically, a solution of lactoyl lactic acid in tert-butyl acetate is added to a solution of perchloric acid, followed by stirring. Then, tert-butyl acetate is further added when necessary. After the completion of the reaction, tert-butyl lactoyl lactate is obtained by common purification and isolation. At the same time, tert-butyl tert-butoxy lactoyl lactate is also obtained.

A solution of the tert-butyl tert-butoxy lactoyl lactate in acetonitrile is added with hydrofluoric acid, followed by stirring at room temperature. After the completion of the reaction, tert-butyl lactoyl lactate can be obtained by common purification and isolation.

By reacting a compound represented by the formula (2) having a protected hydroxyl group and a compound represented by the formula (3) having a protected carboxyl group which are obtained respectively as described above, a compound represented by the formula (1) having a longer chain length can be produced.

This esterification reaction will be described below.

Firstly, to a solution of the compound represented by the formula (2) having the protected hydroxyl group (for example, t-butyldimethylsiloxylactoyl lactic acid) in methylene chloride is added a solution of a compound represented by the formula (3) having the protected carboxyl group (for example, t-butyl lactate) in methylene chloride, and further a solution of 4-dimethylaminopyridine in methylene chloride is added thereto. Temperature is down to 0 °C on an ice bath, followed by starting stirring, and then a solution of N,N'-dicyclohexyl carbodiimide in methylene chloride is added thereto. After stirring, the temperature is returned to room temperature, followed by additional stirring for 3 hours. After the reaction, a compound represented by the formula (1) (t-butyldimethylsiloxy lactic acid trimer t-butyl ester, when t-butyldimethylsiloxylactoyl lactic acid is reacted with tert-butyl lactate) can be obtained by common purification and isolation.

In the lactic acid oligomer represented by the formula (1) obtained as described above, a hydroxyl group and a carboxyl group are both protected.

In the present invention, the compound represented by the formula (1) having a protected hydroxyl group and a protected carboxyl group can be treated with hydrofluoric acid to deprotect selectively the hydroxyl group.

Specifically, to a solution of the compound represented by the formula (1) having a protected hydroxyl group and a protected carboxyl group (for example, t-butyldimethylsiloxy lactic acid trimer tert-butyl ester, or t-butyldimethylsiloxy lactic acid tetramer tert-butyl ester, etc.) is added hydrofluoric acid, followed by stirring. After the reaction, a compound whose hydroxyl group is selectively deprotected can be obtained by a common purification and isolation.

Further, in the present invention, the compound represented by the formula (1) having a protected hydroxyl group and a protected carboxyl group can be treated with trifluoroacetic acid/methylene chloride to deprotect selectively the carboxyl group.

Specifically, to a solution of the compound represented by the formula (1) having a protected hydroxyl group and a protected carboxyl group (for example, t-butyldimethylsiloxy lactic acid tetramer t-butyl ester) in methylene chloride is added a mixed solution of trifluoroacetic acid and methylene chloride, followed by stirring at room temperature. After the completion of the reaction, a compound whose carboxyl group is selectively deprotected can be obtained by a common purification and isolation.

In the method of the present invention, desired compounds represented by the formula (2) or the formula (3) can be obtained by protecting or deprotecting selectively the hydroxyl group or the carboxyl group of a lactic acid oligomer ranging from a trimer to an eicosamaer which is produced by the above method. Then, these compounds can be used as the starting material in the succeeding reaction to produce a lactic acid oligomer having a longer chain length.

The present invention will be described more specifically by the following Examples, but the present invention is not limited to the examples.

### EXAMPLES

### Example 1: Production of tert-butyl 2-hydroxypropionate

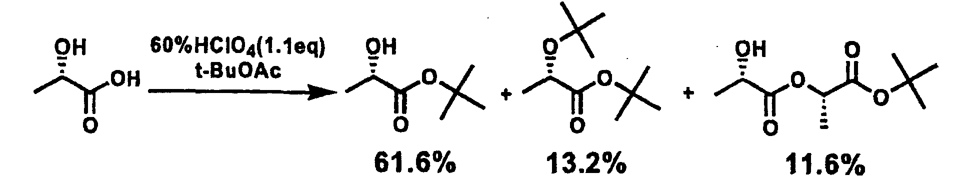

A solution (20 ml) of 2-acetoxypropionic acid (0.4505 g (5 mmol)) in methylene chloride was put in a 50 ml argon-substituted two-neck eggplant-shaped flask. A solution (10ml) of tert-butyl acetate (0.81 ml (6 mmol)) in methylene chloride was added thereto, and then, 60% perchloric acid (0.6 ml) was added, followed by stirring at 35 °C for 4 hours. Further, a solution of tert-butyl acetate (concentration:2.7 mol/l) in methylene chloride was added dropwise over more than 1 hour to allow unreacted 2-acetoxypropionic acid to react. Diethyl ether (30 ml) was added thereto, and a saturated sodium hydrogen carbonate solution (65 ml) was added to stop the reaction. The resultant was extracted three times with diethyl ether (100 ml), dried over anhydrous magnesium sulfate day and night, concentrated by a rotary evaporator while cooling at 8 °C, and isolated by column chromatography (developing solvent: hexane:diethyl ether=3:1) to give tert-butyl 2-hydroxypropionate as a white needle solid at a yield of 61.6%.

### Example 2: Production of tert-butyl lactoyl lactate

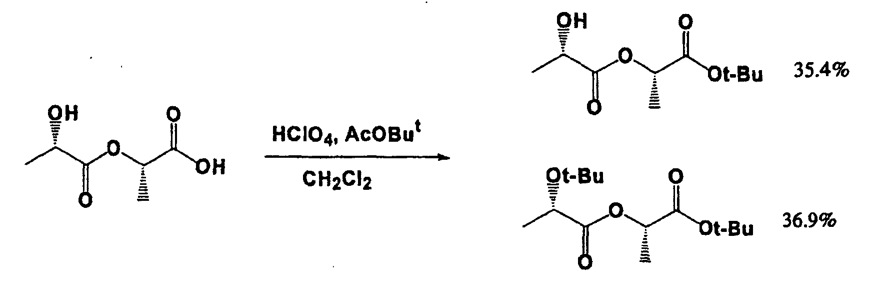

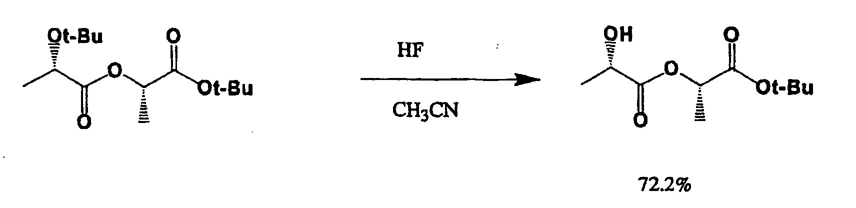

A 60% perchloric acid solution (3.24 ml(27.8 mmol)) was put in a 100 ml argon-substituted two-neck eggplant-shaped flask. A solution (5 ml) of lactoyl lactic acid (4.10 g (25.3 mmol)) in tert-butyl acetate was added thereto, followed by starting stirring and then dropping tert-butyl acetate (25 ml) over more than 30 minutes. The stirring was stopped at 40 minutes after the end of dropping, and a saturated sodium hydrogen carbonate solution (200 ml) was added for neutralization. The resultant was extracted three times with diethyl ether (250 ml), concentrated to one-quarter in volume, dried over anhydrous magnesium sulfate day and night, concentrated, and isolated by column chromatography (developing solvent: hexane:diethyl ether=2:1) to obtain tert-butyl lactoyl lactate (1.9552 g )(yield: 35.4%) as a white oil. Simultaneously, tert-butyl tert-butoxylactoyl lactate (2.5596 g) (yield: 36.9%) was obtained.

To a solution of tert-butyl tert-butoxylactoyl lactate (0.0766 g) (0.2792 mmol) in acetonitrile (5 ml) was added 2 drops of hydrofluoric acid, followed by stirring for 2 hours at room temperature. A saturated sodium hydrogen carbonate solution (5 ml) was added thereto for neutralization, and then a saturated brine (20 ml) was added. The resultant was extracted three times with diethyl ether (25 ml), dried over anhydrous magnesium sulfate day and night, concentrated, and isolated by column chromatography (developing solvent: hexane:diethyl ether=2:1) to obtain tert-butyl lactoyl lactate (0.0440 g) (yield: 72.2%) as a colorless oil.
H NMR (300MHz, CDCl₃)
δ (ppm)=1.44-1.49 (m, 15H)
2.96 (d, 1H, J=6.7Hz)
4.33 (dq, 1H, J=6.7Hz)
5.00 (q, 1H, J=7.2Hz)

### Example 3: Production of t-butyldimethylsiloxy lactoyl acetic acid

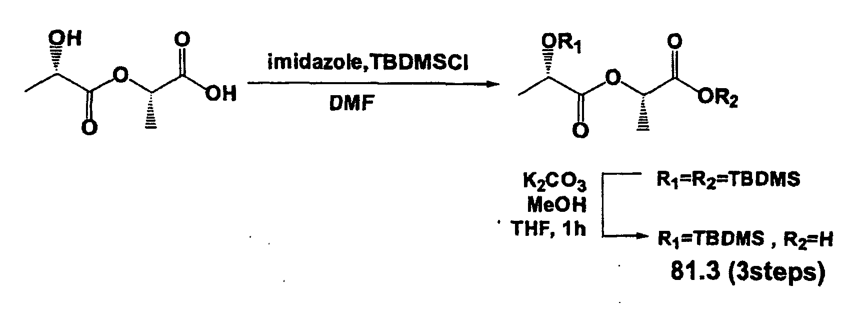

A solution (27 ml) of lactoyl lactic acid (10 mmol) (obtained by ring-opening lactide (10 mmol)) in DMF was put in a 100 ml argon-substituted two-neck eggplant-shaped flask. A solution (23 ml) of t-butyldimethylchlorosilane (TBDMSSCl, 50 mmol) and imidazole (100 mmol) in DMF was added thereto, followed by starting stirring, and the mixture was stirred for 3 hours from the end of dropping. After the completion of the reaction, a saturated sodium chloride solution (250 ml) was added thereto. The resultant was extracted three times with diethyl ether (250 ml), washed with 1N hydrochloric acid (150 ml) cooled at 0 °C, further washed twice with a saturated sodium chloride solution (100 ml), dried over anhydrous magnesium sulfate day and night, concentrated, and isolated by column chromatography (developing solvent: hexane:diethyl ether=1:1) to obtain t-butylsiloxylactoyl lactic acid tert-butylsiloxy ester (5.3881 g) as a colorless oil.

Further, t-butyldimethylsiloxylactoyl lactic acid tert-butyldimethylsiloxy ester (5.3881 g) was dissolved in methanol (100 ml) and THF (33 ml), and the obtained solution was put in a 200 ml argon-substituted two-neck eggplant-shaped flask. A solution of potassium carbonate (3.333 g) in water (33 ml) was dropped thereto, followed by stirring for 1 hour at room temperature. The resultant was concentrated to one-quarter in volume, and a saturated brine (100 ml) was added thereto. 1N potassium hydrogen carbonate was added to adjust the pH of water layer to pH4-5. The product was extracted three times with diethyl ether (150 ml), further washed twice with a saturated sodium chloride solution (50 ml), dried over anhydrous magnesium sulfate day and night, concentrated, and isolated by column chromatography (developing solvent: hexane:diethyl ether=3:1) to obtain tert-butyldimethylsiloxylactoyl lactic acid (2.2458 g) (yield: 81.3%) as a colorless oil.
H NMR (300MHz, CDCl₃)
d (ppm)=0.097 (t, 6H), 0.904 (s, 9H)
1.45 (d, 3H, J=7.0Hz)
1.55 (d, 3H, J=7.0Hz)
4.39 (q, 1H, J=6.7Hz)
5.12 (q, 1H, J=7.1Hz)
8.3-8.5 (broad, 1H)

### Example 4: Production of t-butyldimethylsiloxy lactic acid trimer tert-butyl ester

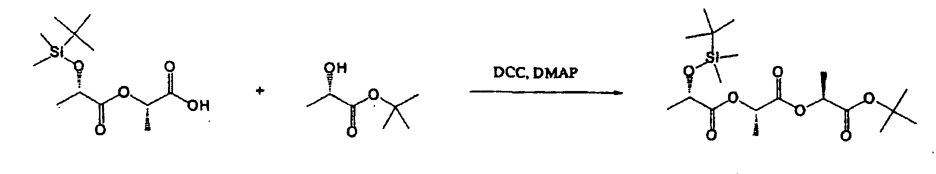

A solution (1 ml) of t-butyldimethylsiloxylactoyl lactic acid (0.2164 g) (0.7829 mmol) in methylene chloride was put in a 30 ml argon-substituted two-neck eggplant-shaped flask. A solution (1 ml) of tert-butyl lactate (0.2289 g) (1.5658 mmol) in methylene chloride was added thereto. A solution (1 ml) of 4-dimethylaminopyridine (0.0973 g) (0.7830 mmol) in methylene chloride was added thereto, followed by starting stirring at 0 °C on an ice bath. A solution (1 ml) of N,N'-dicyclohexylcarbodiimide (0.2112 g) (1.0180 mmol) in methylene chloride was added thereto, followed by rinsing with methylene chloride (11 ml) which was also poured. After stirring for 5 minutes, the mixture was returned to room temperature, followed by stirring for additional 3 hours. The product was filtered by an aspirator, and a saturated ammonium chloride solution (15 ml) was added thereto to stop the reaction, followed by neutralizing with a saturated sodium hydrogen carbonate (16 ml). The resultant was extracted three times with diethyl ether (50 ml), dried over anhydrous magnesium sulfate for 2 hours, concentrated, and isolated by column chromatography (developing solvent: hexane:diethyl ether=3:1) to obtain t-butyldimethylsiloxy lactic acid trimer tert-butyl ester as a colorless and transparent oily substance at a yield of 72.8%.
H NMR (300MHz, CDCl₃)
d (ppm)=0.12 (d, 6H, J=7.5Hz)
0.91 (s, 9H)
1.44-1.49 (m, 15H)
1.58 (d, 3H, J=7.2Hz).
4.40 (q, 1H, J=6.8Hz)
5.00 (q, 1H, J=7.1Hz)
5.14 (q, 1H, J=7.0Hz)

### Example 5: Selective deprotection of the trimer

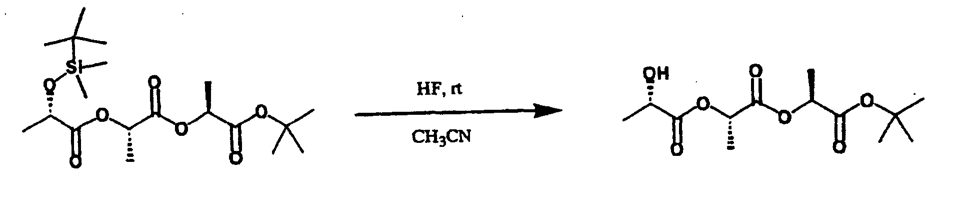

A solution of t-butyldimethylsiloxy lactic acid trimer tert-butyl ester (0.0969 g) (0.2397 mmol) in THF (1 ml) was put in a 30 ml argon-substituted two-neck eggplant-shaped flask, and acetic acid (3 ml) and water (1 ml) were added thereto, followed by stirring for 3 hours at room temperature. No change was found. Then, two drops of hydrofluoric acid were added thereto, followed by stirring for 2 hours and then adding a saturated sodium hydrogen carbonate solution (50 ml). The resultant was extracted three times with diethyl ether (100 ml), dried over anhydrous magnesium sulfate day and night, concentrated, and isolated by column chromatography (developing solvent: hexane:diethyl ether=2:1) to obtain lactic acid trimer tert-butyl ester (0.0592 g) (yield: 85.2%) as a colorless oil.
H NMR (300MHz, CDCl₃)
d (ppm)=1.43 (s, 9H)
1.46 (d, 3H, J=6.9Hz)
1.46 (d, 3H, J=7.2Hz)
1.47 (d, 3H, J=7.2Hz)
2.95-3.05 (broad, 1H)
4.34 (q, 1H, J=6.9Hz)
4.97 (q, 1H, J=6.9Hz)
5.19 (q, 1H, J=6.9Hz)

### Example 6: Production of t-butyldimethylsiloxy lactic acid tetramer tert-butyl ester

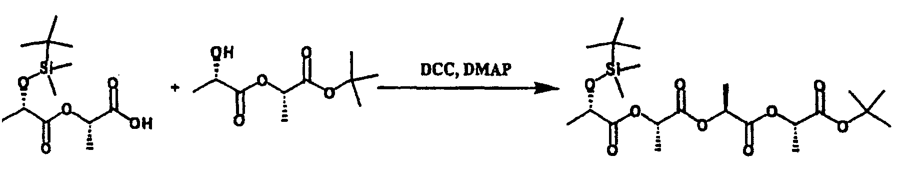

A solution (5 ml) of t-butyldimethylsiloxylactoyl lactic acid (2.5623 g) (9.2703 mmol) in methylene chloride was put in a 100 ml argon-substituted two-neck eggplant-shaped flask. A solution (5 ml) of tert-butyl lactoyl lactate (1.8892 g) (8.6561 mmol) in methylene chloride was added thereto. A solution (10 ml) of 4-dimethylaminopyridine (1.0759 g) (8.6561 mmol) in methylene chloride was added thereto, and the mixture was cooled to 0 °C on an ice bath, followed by stirring. A solution (15 ml) of N,N'-dicyclohexylcarbodiimide (4.0975 g) (15.581 mmol) in methylene chloride was added thereto, followed by stirring for 5 minutes. The resultant was returned to room temperature, followed by stirring for additional 2 hours. The product was filtered by an aspirator, and a saturated ammonium chloride solution (140 ml) was added thereto to stop the reaction, followed by neutralizing with a saturated sodium hydrogen carbonate (150 ml). The resultant was extracted three times with diethyl ether (200 ml), concentrated to a volume of about 100 ml, dried over anhydrous magnesium sulfate for 2 hours, concentrated, and isolated by column chromatography (developing solvent: hexane:diethyl ether=3:1) to obtain t-butyldimethylsiloxy lactic acid tetramer tert-butyl ester as a white powdery solid at a yield of 76.8%.
H NMR (300MHz, CDCl₃)
d (ppm)=0.10 (d, 6H, J=7.2Hz)
0.90 (s, 9H)
1.44-1.48 (m, 15H)
1.58 (d, 6H, J=6.9Hz)
1.59 (d, 3H, J=6.8Hz)
4.40 (q, 1H, J=6.8Hz)
4.98 (q, 1H, J=7.0Hz)
5.12 (q, 1H, J=7.2Hz)
5.18 (q, 1H, J=7.2Hz)

### Example 7: Selective deprotection of the tetramer

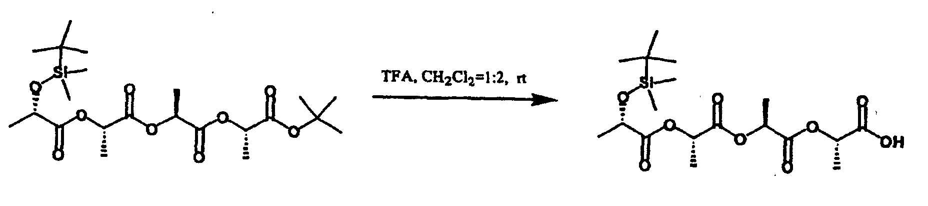

To a solution of t-butyldimethylsiloxy lactic acid tetramer tert-butyl ester (0.2011 g) (0.4223 mmol) in methylene chloride (10ml) was dropped a mixed solution of trifluoroacetic acid (10 ml) and methylene chloride (10 ml), followed by stirring at room temperature for 40 minutes after the end of dropping. A saturated sodium hydrogen carbonate was added to adjust the pH of water layer to pH 9, and then 1N hydrochloric acid cooled to 0 °C was dropped thereto to adjust the pH of water layer to pH 3-4. The resultant was extracted three times with diethyl ether (100 ml), washed with a saturated aqueous sodium chloride solution (100 ml), dried over anhydrous magnesium sulfate day and night, concentrated, and isolated by column chromatography (developing solvent: hexane:diethyl ether=1:1) to obtain t-butyldimethylsiloxy lactic acid tetramer (0.1038 g) (yield:84.1%) as a pale yellow oil.
H NMR (300MHz, CDCl₃)
d (ppm)=0.10 (d, 6H, J=6.9Hz)
0.91 (s, 9H), 1.46 (d, 3H, J=6.9Hz), 1.56 (d, 3H, J=6.9Hz), 1.60 (d, 3H, J=6.9Hz), 4.41 (q, 1H, J=6.6Hz), 5.13 (q, 1H, J=7.3Hz), 5.17 (q, 1H, J=7.1Hz)

### Example 8: Selective deprotection of the tetramer

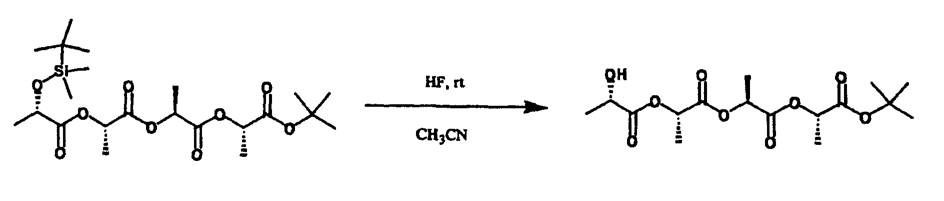

To a solution of t-butyldimethylsiloxy lactic acid tetramer tert-butyl ester (1.0009 g) (2.1000 mmol) in acetonitrile (25 ml) was added five drops of hydrofluoric acid, followed by stirring to be continued for 1 hour at room temperature. A saturated sodium hydrogen carbonate solution (15 ml) was added for neutralization, and then a saturated brine (60 ml) was added thereto. The resultant was extracted three times with diethyl ether (100 ml), dried over anhydrous magnesium sulfate day and night, concentrated, and isolated by column chromatography (developing solvent: hexane:diethyl ether=1:2) to obtain lactic acid tetramer tert-butyl ester (0.6982 g) (yield:91.8%) as a colorless oil.
H NMR (300MHz, CDCl₃)
d (ppm)=0.10 (d, 6H, J=7.2Hz), 0.90 (s, 9H)
1.45 (s, 9H), 1.47 (d, 3H, J=6.8Hz)
1.49 (d, 3H, J=7.2Hz), 1.61 (d, 6H, J=6.8Hz)
4.40 (q, 1H, J=6.8Hz), 2.74 (d, 1H, J=5.7Hz)
4.36 (dq, 1H, J=6.0Hz)
4.99 (q, 1H, J=6.8Hz), 5.19 (q, 1H, J=7.1Hz)
5.22 (q, 1H, d=7.2Hz)

### Example 9: Selective deprotection of the tetramer

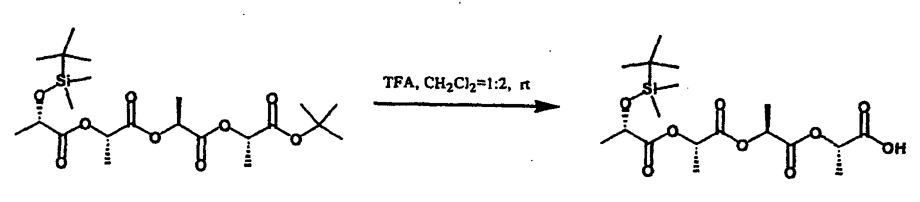

To a solution of TBDMS ether tert-butyl ester of lactic acid tetramer (0.2373 g) (0.4982 mmol) in methylene chloride (5 ml) was dropped trifluoroacetic acid (2.5 ml), followed by stirring at room temperature for 1 hour after the end of dropping. A saturated sodium hydrogen carbonate solution (34 ml) was added to adjust the pH of water layer to pH 8, and then seven drops of 1N hydrochloric acid cooled to 0 °C were dropped thereto to adjust the pH of water layer to pH 4. The resultant was extracted three times with diethyl ether (75 ml), dried over anhydrous magnesium sulfate day and night, concentrated, and isolated by column chromatography (developing solvent: hexane:diethyl ether=1:1) to obtain lactic acid tetramer TBDMS ether (0.1720 g) (yield:82.1%) as a pale yellow oil.
H NMR (300MHz, CDCl₃)
d (ppm)=0.09 (d, 6H, J=7.2Hz)
0.89 (s, 9H)
1.44 (d, 3H, J=6.9Hz)
1.54 (d, 6H, J=7.2Hz)
1.56-1.58 (m, 6H,)
4.39 (q, 1H, J=6.9Hz)
5.09-5.21 (m, 3H)

### Example 10: Selective deprotection of the tetramer

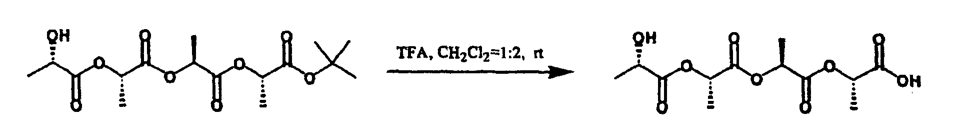

To a solution of lactic acid tetramer tert-butyl ester (0.6982 g) (1.9268 mmol) in methylene chloride (25 ml) was dropped a mixed solution of trifluoroacetic acid (2.5 ml) and methylene chloride (2.5 ml), followed by stirring at room temperature for 1 hour after the end of dropping. A saturated sodium hydrogen carbonate solution (30 ml) was added to adjust the pH of water layer to pH 8, and then a saturated ammonium chloride (50 ml) was added thereto to adjust the pH of water layer to pH 6. The resultant was extracted three times with diethyl ether (100 ml). The extraction solution contained almost all impurities and a small amount of the substance of interest.

To the remaining water layer was dropped 1N hydrochloric acid (5 ml) cooled at 0 °C, so as to adjust the pH of water layer to pH 2-3. The layer was extracted three times with methylene chloride (150 ml). At this time, the pH changed, and therefore 1N hydrochloric acid cooled at 0°C was used to keep the pH of the water layer at pH 2-3. The resultant was dried over anhydrous magnesium sulfate day and night, concentrated, and isolated by column chromatography (developing solvent: hexane:diethyl ether=1:4) to obtain lactic acid tetramer (0.2047 g) (yield:34.7%) as a colorless oil.

### Example 11: Production of t-butyldimethylsiloxy lactic acid hexamer tert-butyl ester

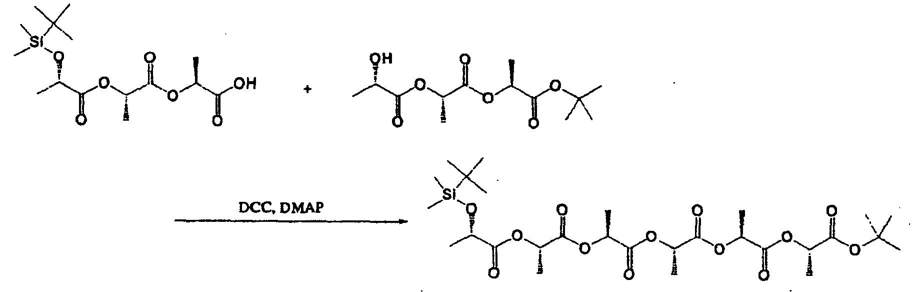

A solution (2.5 ml) of t-butyldimethylsiloxylactoyl lactic acid trimer (0.0880 g) (0.2500 mmol) in methylene chloride was put in a 30ml argon-substituted two-neck eggplant-shaped flask. A solution (2.5 ml) of lactic acid trimer tert-butyl (0.0306 g) (0.1054 mmol) in methylene chloride was added thereto, followed by starting stirring after cooled to 0 °C on an ice bath. A solution (2.5 ml) of 4-dimethylaminopyridine (0.0130g) (mmol) in methylene chloride was added thereto, and then a solution 2.5 ml of N,N'-dicyclohexylcarbodiimide (0.0282 g) (mmol) in methylene chloride was added thereto. After stirring for 5 minutes, the resultant was returned to room temperature, followed by stirring for additional 3 hours. The product was filtered by an aspirator, and a saturated ammonium chloride solution (2.5 ml) was added thereto to stop the reaction, followed by neutralizing with a saturated sodium hydrogen carbonate (3 ml). The resultant was extracted three times with diethyl ether (50 ml), dried over anhydrous magnesium sulfate for 2 hours, concentrated, and isolated by column chromatography (developing solvent: hexane:diethyl ether=3:1) to obtain t-butyldimethylsiloxy lactic acid hexamer tert-butyl ester a pale yellow solid at a yield of 72.5%.
H NMR (300MHz, CDCl₃)
d (ppm)=0.089 (d, 6H, J=6.9Hz)
0.89 (s, 9H)
1.43-1.47 (m, 15H)
1.56-1.59 (m, 12H)
4.39 (q, 1H, J=6.9Hz)
4.97 (q, 1H, J=7.1Hz)
5.08-5.20 (m, 4H)

### INDUSTRIAL APPLICABILITY

The present invention makes it possible to synthesize an oligolactic acid ester having a specific chain length as a single compound. Utilization of the single compound of oligolactic acid ester which is provided by the present invention allows the oligolactic acid ester to be developed as a drug, a drug raw material, a food additive, a cosmetic raw material, a pharmaceutic raw material, and a pharmaceutic additive.

## Claims

1. A method for producing a compound represented by formula (1): 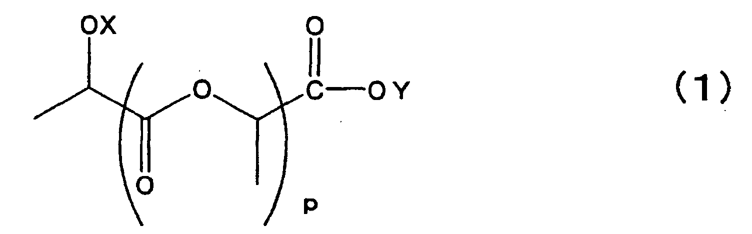 (in the formula (1), X represents a hydrogen atom or a protective group for a hydroxyl group, Y represents a hydrogen atom or a lower alkyl group, and p represents an integer of 2 to 19, satisfying m+n+1=p),
which comprises reacting a compound represented by formula (2) with a compound represented by formula (3): 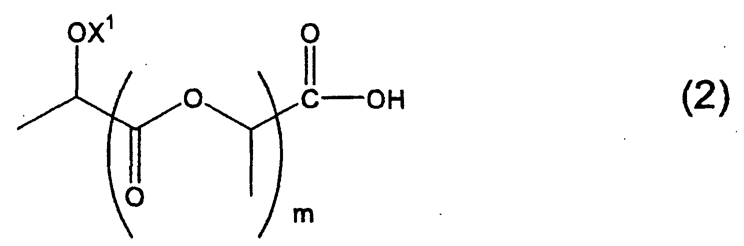 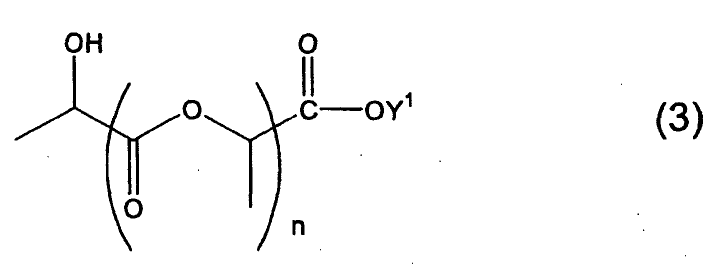 (in the formula (2), X¹ represents a protective group for a hydroxyl group, and m represents an integer of 0-18; and in the formula (3), Y¹ represents a lower alkyl group, and n represents an integer of 0-18).

2. A method for deprotecting selectively a protective group for a hydroxyl group in the formula (1): 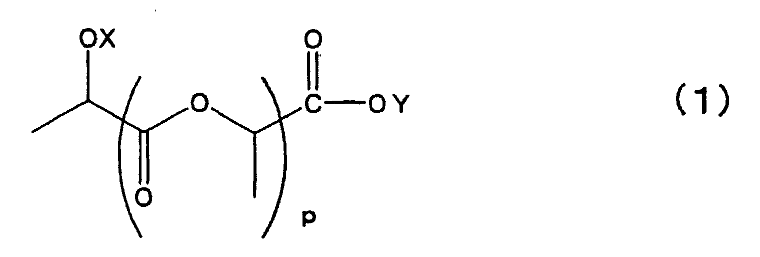 wherein X represents a protective group for a hydroxyl group, Y represents a lower alkyl group, and m represents an integer of 1 to 19,
wherein the compound represented by the formula (1) is treated with hydrofluoric acid.

3. A method for deprotecting selectively a protective group for a carboxyl group in the formula (1): 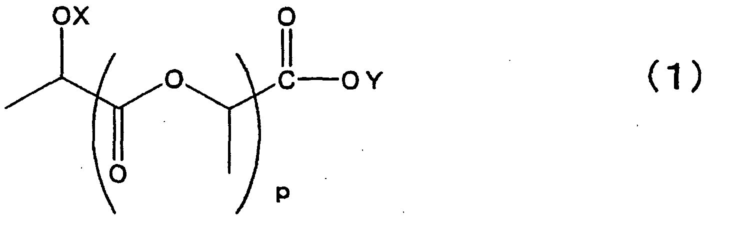 wherein X represents a protective group for a hydroxyl group, Y represents a lower alkyl group, and m represents an integer of 1 to 19,
wherein the compound represented by the formula (1) is treated with trifluoroacetic acid/methylene chloride.

4. The method according to any of claims 1 to 3, wherein X is a trialkylsilyl group.

5. The method according to any of claims 1 to 4, wherein Y is a tert-butyl group.
